# EUROPÄISCHE PATENTSCHRIFT

(11) **EP 0 711 113 B1**
(45) Veröffentlichungstag und Bekanntmachung des Hinweises auf die Patenterteilung: **23.07.1997**
(21) Anmeldenummer: 95906894.1
(22) Anmeldetag: 01.02.1995
(51) Int. Cl.: A01N 59/16, A61L 29/00, C08K 3/10

(54) **VERFAHREN ZUR HERSTELLUNG VON BAKTERIZIDEN/FUNGIZIDEN KUNSTSTOFFKÖRPERN**
PROCESS FOR PRODUCING BACTERICIDAL/FUNGICIDAL PLASTIC BODIES
PROCEDE DE FABRICATION DE CORPS PLASTIQUES BACTERICIDES/FONGICIDES

(30) Priorität: 01.02.1994 DE 4403016; 05.08.1994 DE 4427829
(43) Veröffentlichungstag der Anmeldung: 15.05.1996
(73) Patentinhaber: KRALL, Theodor, A-6600 Lechaschau (AT); Guggenbichler, J. Peter, D-96049 Bamberg (DE)
(72) Erfinder: KRALL, Theodor, A-6600 Lechaschau (AT); Guggenbichler, J. Peter, D-96049 Bamberg (DE)
(74) Vertreter: VOSSIUS & PARTNER
(86) Internationale Anmeldenummer: DE9500122
(87) Internationale Veröffentlichungsnummer: WO9520878

(56) Entgegenhaltungen:
- EP-A- 0 433 961
- WO-A-93/23092
- DE-A- 3 837 125

## Beschreibung

Die Erfindung betrifft Verfahren zur Herstellung von weiterverarbeitungsfähigen Kunststoffkörpern insbesondere für die Herstellung von Gegenständen für den medizinischen Bedarf, die einen antimikrobiell wirksamen Gehalt an Metallen oder Metallverbindungen (im folgenden Wirkstoffe genannt) aufweisen.

Hierunter sind insbesondere solche Metalle bzw. deren Verbindungen zu verstehen, deren oligodynamische Wirkung bekannt ist, wie z.B. Silber, Kupfer und Gold, aber auch andere Schwermetalle wie z.B. Zink und auch Lanthanide, die auf Bakterien oder/und Pilze im erfindungsgemäß gewünschten Sinn einwirken, d.h. sie abtöten, deren Vermehrung oder auch deren Haftvermögen am oder Einnistungsvermögen im Kunststoff verhindern oder zumindest stark vermindern.

Derartige Vorprodukte oder Fertigteile aus derartigen Kunststoffen sind nach dem heutigen Stand der Marktlage zwar noch nicht allgemein erhältlich, nach dem Stand der Technik aber durchaus funktionsfähig herstellbar.

Ein Grund, daß die Markteinführung noch nicht in nennenswertem Umfang erfolgt ist, dürfte die Frage des Aufwandes und damit der Kosten für die Herstellung derartiger Kunststoffe sein.

Das betrifft einerseits die Kosten der für den angestrebten Zweck notwendigen Mengen des Metalles bzw. der Metallverbindungen, insbesondere von Silber, wenn diese Substanzen in pulvriger Form in den Kunststoff eingearbeitet werden sollen, wobei als untere Wirksamkeitsgrenze immer wieder die Größenordnung von 1 Gewichts-% des Kunststoffes genannt wird, größere Anteile aber stets als noch wirkungsvoller angegeben werden. In diesem Zusammenhang sei auf die Patentveröffentlichungen US-A-4 054 139, WO-A-84/01721; EP-A-0 190 504, DE-A-37 25 728, EP-A-0 251 783 und DE-A-39 42 112 verwiesen.

Das betrifft andererseits die Kosten für das letztlich doch recht aufwendige und dementsprechend zweckmäßigerweise nur für ausgewählte Fälle anzuwendende, nasse Verfahren der Behandlung der Kunststoffe z.B. gemäß DE-C-42 26 810, gemäß welchem sehr geringe Mengen des Wirkstoffs ausreichend sind.

Eine andere Möglichkeit, die hohen Materialkosten für die antimikrobielle Ausrüstung von Gegenständen zu vermeiden, besteht darin, nicht den Kunststoff im ganzen antimikrobiell auszurüsten, sondern die aus diesem Kunststoff hergestellten Gegenstände nachträglich mit Wirkstoffen zu beschichten.

Jedoch funktionieren aber alle physikalischen Verfahren (wie z.B. Bedampfung, Kathodenzerstäubung, plasmaunterstützte Bedampfung, Ionenplattierung, Ionenimplantation) und auch die chemischen Verfahren (z.B. stromlose Galvanisierung, reaktives Bedampfen, reaktive Kathodenzerstäubung, CVD, PACVD) so, daß damit nur die der jeweiligen Quelle der aufzubringenden Wirksubstanz zugewandten Flächen oder, z.B. im Fall von plasmaunterstützen Verfahren, zumindest jeweils nur die der Umgebung offen zugewandten Flächen beschichtet werden. Die für medizinische Anwendungen meist besonders wichtigen Innenflächen von Gegenständen, z.B. die Innenseite von Kathetern sind für die Anwendung dieser vorgenannten Verfahren aber unzugänglich und bleiben daher unbeschichtet.

Die Aufgabe der vorliegenden Erfindung ist somit die Bereitstellung eines Verfahrens zur Herstellung von oligodynamisch wirksamen Kunststoffkörpern, welche die vorstehenden Nachteile nicht aufweisen, d.h. einfach herstellbar sind, nur eine geringe Menge des oligodynamischen Metalls bzw. dessen Verbindung(en) benötigen und an allen, auch unzugänglichen Oberflächen gleich wirksam sind.

Gelöst wird diese Aufgabe durch ein Verfahren, mit dem Kunststoffkörper hergestellt werden können, die einen Gehalt an einem oder mehreren oligodynamisch wirksamen Metallen oder Metallverbindungen als Wirkstoff haben, die dadurch gekennzeichnet sind, daß der Wirkstoff in Form von diskreten Teilchen im Kunststoff eingebettet ist, wobei die Wirkstoffmenge nicht mehr als 1,0 Gew.-%, bevorzugt nicht mehr als 0,5 Gew.-%, bezogen auf das Gesamtgewicht des Kunststoffkörpers, beträgt und das Maximum der diskreten Teilchengröße des Wirkstoffs unterhalb von 500 nm liegt.

Gut hantierbare Wirkstoffe jeglicher Art in Pulverform werden nur mit Kornfeinheiten bis in den Mikronbereich (d.h. ≥ 1 µm) und Submikronbereich (d.h. > 0,1 µm) hergestellt und angeboten. Die damit erreichte, spezifische Oberfläche beträgt beispielsweise für das feinste handelsübliche Silberpulver mit einer Nenn-Korngröße von 2 - 3,5 µm, das natürlich auch einen gewissen, verfahrensbedingt unvermeidlichen Anteil von gröberen und von Sub-Mikron-Partikeln aufweist, nach der Angabe des Herstellers etwa 0,5 bis 1 m²/g. Andere, häufig verwendete und auch billigere Silberpulver haben noch gröbere Partikel und dementsprechend geringere spezifische Oberflächen.

Noch feinere, kolloidale Zubereitungen können im allgemeinen nur als Sole oder Gele dargestellt werden. Dabei ist der also gegebene Anteil an Schutzkolloid in der weiteren Folge der Verarbeitung und der Anwendung meist mit erheblichen, unerwünschten Nebenwirkungen verbunden. Außerdem sind derartige kolloidale Zubereitungen oft wenig stabil und zudem häufig verhältnismäßig teuer.

Bei dem erfindungsgemäßen Verfahren werden Kunststoffkörper deshalb vorzugsweise so hergestellt, daß man den Rohling mittels eines chemischen oder physikalischen Verfahrens mit der bakterizid und/oder fungizid (oligodynamisch) wirksamen Substanz beschichtet, den erhaltenen beschichteten Rohling (Vorprodukt) zerkleinert und/oder einschmilzt, woraus dann nach üblichen Verfahren der gewünschte Kunststoffkörper hergestellt wird.

Mit physikalischen, aber auch mit chemischen Verfahren der Beschichtungen von Oberflächen können sehr dünne und, je nach Prozeßführung, feine oder sogar äußerst feinstrukturierte Gefüge des abgeschiedenen Materials erzielt werden, die der Feinheit kolloidaler Zubereitungsformen entsprechen oder wenigstens nahekommen. Das gilt insbesondere dann, wenn die abgeschiedenen Schichten sehr dünn sind.

Bei solchen Beschichtungen ergibt sich z.B. schon rein rechnerisch aus der äußeren Geometrie bei einer Schichtdicke von 10 nm bereits z.B. für die Abscheidung von Silber ein Mindestwert der spezifischen Oberfläche von 19 m²/g. Vorzugsweise beträgt die Schichtdicke 1 bis 50 nm.
Werden dabei noch zusätzlich jene Oberflächen berücksichtigt, die senkrecht zur Hauptfläche durch noch offen daliegende Korngrenzen und durch Zerklüftungen der Oberfläche durch Fehler der Gleichmäßigkeit der Abscheidung entstehen, ergibt sich für die spezifische Oberfläche praktisch ein noch größerer Wert.

Vorzugsweise liegt das Maximum der Teilchengrößenverteilung des Wirkstoffs in mindestens einer Dimension (z.B. bei Teilchen, die in Form von Flocken oder Flitter vorliegen) unterhalb von 100 nm, stärker bevorzugt bei 10 nm. Bei dünnen Schichten kann die Korngröße auch kleiner als 1 nm sein.

Kristalline Materialien (PTFE, manche Polyimide) müssen z.B. durch Mahlen zerkleinert werden; Umschmelzen ist dabei (praktisch) nicht anzuwenden. Sie werden dann durch z.B. (Druck-) Sintern in die gewünschte Form gebracht.

Es hat sich bei den Versuchen gezeigt, daß Kunststoffe mit einem an sich geringen, aber feinst dispergiertem Gehalt von Metallpartikeln (oder auch Partikeln aus Metallverbindungen) mit dementsprechend großer spezifischer, aktiver Oberfläche in der Wirkung gleichzusetzen sind mit Kunststoffen, die einen wesentlich höheren, aber weniger fein dispergierten Gehalt an Metallen (oder auch Metallverbindungen) mit dementsprechend kleinerer spezifischer, aktiver Oberfläche aufweisen.

Da unbestreitbar aber der für die Langzeitwirkung maßgebliche Vorrat von diesen Wirksubstanzen bei gröberen Partikeln größer ist, kann es als weiterer Vorteil von nach dem vorstehend genannten Verfahren hergestellten Kunststoffen angeführt werden, daß diese Kunststoffe für besonders kritische Anwendungsfälle durch geeignete Prozeßführung bei der Herstellung der Schicht von Metall (oder von Metallverbindungen) mit Partikeln ausgerüstet werden können, deren Größe für eine optimale Langzeitwirkung von vorneherein beeinflußbar ist.

Die Beschichtung kann auf Folien erfolgen (siehe Beispiel), die dann wieder zerkleinert und weiterverarbeitet werden können. Sie kann aber auch auf Fasern oder auf Granulat, mit demselben Ergebnis der Herstellung der gewünschten Endkonzentration des Wirkstoffes im Kunststoff aufgebracht werden.

Als Kunststoffrohlinge können auch solche verwendet werden, in die bereits Füllstoffe eingemischt sind. Bevorzugt werden dabei Kunststoffrohlinge, deren Füllstoffe die chemischen oder physiologischen Eigenschaften des als Vorprodukt verwendeten Kunststoffes nicht wesentlich verändern. Als Füllstoffe werden solche bevorzugt, die die röntgentechnische Erkennbarkeit und/oder die Erhöhung des spezifischen Gewichtes von daraus hergestellten Endprodukten (z.B. Schürzen) bewirken; im Stand der Technik wird dazu häufig Bariumsulfat verwendet. Kunststoffrohlinge mit Füllstoffen haben außerdem eine größere Oberfläche, was sich günstig bei der erfindungsgemäßen Beschichtung auswirkt (bessere Dispersion).

Die Beschichtung von Kunststoffen in Form von Folien, Bändern (einem Sonderfall von Folien), Fasern oder Granulat mit einem oder mehreren Wirkstoffen in dünnen Schichten, vorzugsweise mit Schichtstärken zwischen 10 und 100 nm, ist auf vielfältige Weise mit chemischen und, vor allem auch, mit physikalischen Verfahren möglich.

Im folgenden wird nicht auf diese Beschichtungstechniken an sich, sondern beispielhaft nur auf die Verwendungsweise solcher mit einer dieser Techniken beschichteten Kunststoffe eingegangen und hierbei im besonderen angenommen, daß die Beschichtung mit physikalischen Verfahren erfolgt.

Alle für die Beschichtung in Betracht gezogenen Verfahren gehen unter Vakuum vor sich und führen damit im Verfahrensschritt der Beschichtung automatisch zu einer Trocknung des zu beschichtenden Kunststoffes, die allen im folgenden noch erwähnten Ansprüchen des erfindungsgemäß gefertigten Kunststoffes bei seiner Weiterverarbeitung genügt.

Ferner wird zur Vereinfachung angenommen, daß die Beschichtung nur mit Metallen im Elementarzustand erfolgt, und daß die beim Beschichtungsvorgang entstehende Metallschicht im allgemeinen sehr dicht und damit in für verarbeitungstechnische Überlegungen relevanten Zeitspannen z.B. für Wasserdampf eine Sperrwirkung aufweist.

Wegen der überragenden Bedeutung dieser Werkstoffgruppe für medizinische Katheter stellt dauer-thermoplastisches Polyurethan ein bevorzugtes Kunststoff-Rohmaterial dar. Den gängigen Polyurethanen ähnliche, neuartige Kunststoffe (z.B. "Carbothane"), seien hier außerdem erwähnt.

Für die Verwendung anderer Materialien müssen die im folgenden beschriebenen Möglichkeiten hingegen materialgerecht und sinngemäß übertragen werden.

Es ist eine besondere Eigenschaft der Polyurethane wie auch der "Carbothane", ziemlich stark hygroskopisch zu sein. Es ist in der Folge dieser Tatsache zu berücksichtigen, daß das Ignorieren von aufgenommener Feuchtigkeit bei Verarbeitungsschritten, bei denen (für Kunststoffe relativ hohe) Temperaturen verwendet werden, zu unerwünschten, die Qualität mindernden und, medizinisch gesehen, gegebenenfalls auch schädlichen Nebenprodukten führen kann. Infolgedessen muß unbedingt verhindert werden, daß der Kunststoff in irgendwelche Verarbeitungsschritte gerät, bei denen solche (für Kunststoffe relativ hohe) Temperaturen auftreten, während er Feuchtigkeit in sich hat.

Ziel der nachfolgend aufgeführten Verfahrensvarianten ist es, den nach der Beschichtung mit Wirkstoff behafteten Kunststoff in eine Form zu bringen, daß dessen Weiterverarbeitung zu Zwischenprodukten in Mischern, Knetern, Extrudern oder auch anderen Maschinen bzw. dessen Endverarbeitung entweder in Extrudern, in Spritzgußmaschinen oder aber auch in anderen Einrichtungen, wie z.B. (Heiß-) Pressen, möglich wird.

In allen diesen Maschinentypen geht die Verarbeitung des eingesetzten dauerthermoplastischen Kunststoffes aber unter der Einwirkung von Temperaturen vor sich, die für Kunststoffe relativ hoch sind. Bei der Beladung solcher Maschinen, besonders Extruder, ist daher mit Rücksicht auf diese Temperatureinwirkung dafür zu sorgen, daß der zu verarbeitende Kunststoff
- entweder seit der, im allgemeinen im Vakuum vorgenommenen, Beschichtung keine Feuchtigkeit mehr aufnehmen konnte,
wobei dies in manchen Fällen bereits dadurch verhindert wird, daß der Kunststoff durch die Beschichtung selbst
- entweder weitgehend (gültig für 2-seitig beschichtete Kunststoff-Folien, je breiter im Verhältnis zu ihrer Dicke, je besser)
- oder vollständig
   (gültig für Kunststoff in der Form von außen beschichtetem Granulat, praktisch gültig auch für Kunststoff-Fasern oder -Bänder)
geschützt wird,
- oder daß der Kunststoff nach dem Beschichtungsvorgang konsequent insoweit er nicht vollständig beschichtet worden ist, durch besondere Maßnahmen gegen den Zutritt von Feuchtigkeit geschützt wird, d.h. aus der Beschichtungsapparatur entnommen, transportiert, gelagert und zum Extruder verbracht wird (Verpackung unter Luftausschluß oder sogar unter Schutzgas)
- oder daß der Kunststoff, der Feuchtigkeit aufgenommen hat (oder haben könnte), vor dem Extrusionsvorgang bevorzugt am Extruder selbst, noch einmal wirksam nachgetrocknet wird.

Zu beachten ist auch, daß in manchen Fällen bereits beschichtetem Kunststoff evtl. unbeschichtete Folien oder Fasern (oder auch unbeschichtetes Granulat) zur Verarbeitung im Extruder beigemischt werden sollen.
Diese müssen dann natürlich ebenfalls unbedingt trocken in den Extruder eingebracht werden, was durch entsprechende Vorkehrungen sicherzustellen ist.

Auf trockenen Zustand zu achten bzw. diesen Zustand herzustellen, ist auch vor dem nächsten, mit Temperatureinwirkung verbundenen Schritt, d.h. wenn bereits beschichteter Kunststoff im letzten Verarbeitungsschritt durchgeknetet worden ist, also den (die) Wirkstoff(e) zwar schon enthält, aber noch nicht durch eine Beschichtung neuerlich versiegelt worden ist, sehr wichtig.

Es sei an dieser Stelle darauf hingewiesen, daß zwar eine Nachtrocknung von Granulat am Extruder stets relativ leicht möglich ist, eine Nachtrocknung von Fasern schon schwieriger ist, eine Nachtrocknung von Folie direkt am Extruder aber Spezialanlagen erfordert, die kaum irgendwo vorhanden sein dürften.

Was die Beladung von Extrudern betrifft, durch die der Kunststoff seine letztendlich gewünschte Form erhält, wird im folgenden davon ausgegangen, daß die Misch- und Knetwirkung im Extruder, welcher der Kunststoff bei der Extrusion unterworfen wird, die erfindungsgemäße Einarbeitung des (der) als Beschichtung(en) aufgebrachten Wirkstoffe(s) in die Kunststoffmasse vollständig homogen bewirkt, wobei unter Umständen Spezialschnecken nötig sind, deren besonders ausgebildete Segmente die aufgetragene Beschichtung feinst zu zerreißen und zu dispergieren in der Lage sind.

Dabei ist es im Prinzip gleichgültig, ob Extruder
- mit (beschichtetem) Granulat, oder
- mittels spezieller Einzugsvorrichtungen mit (schmalen) Bändern oder
- mit (breiten) Folien bzw. Folienpaketen, die 1-seitig oder 2-seitig beschichtet, unter Umständen auch mehrlagig geschichtet bzw. paketiert sein können und dabei auch unbeschichtete (Zwischen-) Folien enthalten können,
- oder aber auch mit Fasern bzw. Faserbündeln
ganz oder vorzerkleinert beladen werden.

In bezug auf Spritzgußmaschinen wird davon ausgegangen, daß die häufig in solchen Maschinen vorhandenen Zuführungsschnecken im allgemeinen nicht jene Misch- und Knetwirkung erbringen, die von Extrudern zu erwarten ist, sondern daß diese Maschinen mit homogen durchmischtem Granulat oder wenigstens gehäckseltem Material beladen werden müssen, um zu befriedigenden Ergebnissen zu gelangen.

Bei Spritzgußmaschinen oder auch (Heiß-)Pressen wird im allgemeinen, zumindest aber bei dauerplastischen Materialien (insbesondere also bei Polyurethan) am besten fertig durchmischter und durchgekneteter Kunststoff in der Form von Granulat zum Einsatz kommen, welches aber wieder Feuchtigkeit aufnehmen kann und daher, wenn es nicht vollständig gegen die Aufnahme von Feuchtigkeit geschützt aus der Vorverarbeitung kommt, direkt vor der Beladung der Maschine, am besten on-line, nachgetrocknet werden muß.

Im speziellen Fall der Verwendung von Folie als Rohmaterial für das erfindungsgemäße Verfahren gilt: Die nach der Weiterverarbeitung aktive Fläche des Wirkstoffes pro Volumeneinheit Kunststoff ist nur abhängig von der, im Zustand nach der Beschichtung, zustandegekommenen Zahl der Lagen Wirkstoff pro Dickeneinheit Kunststoff, d.h. sie ist nur von der Dicke der Folien und davon, ob diese 1-seitig oder 2-seitig beschichtet werden, abhängig. Mit der Dicke der Beschichtung wird lediglich der Wirkstoffvorrat bestimmt, d.h. die Zeit, wie lange das System wirksam bleibt.

Neben der nominellen Schichtdicke haben das gewählte Beschichtungsverfahren und die hierbei verwendeten Parameter, insbesondere auch die innerhalb des Beschichtungsverfahrens für die Vorbehandlung der Oberfläche verwendeten Parameter (z.B. beim Glimmen oder bei der Sputter-Ätzung), großen Einfluß darauf, ob eine hergestellte Schicht nur scheinbar (optisch) oder wirklich gegen Wasserdampf dicht ist.

Letztlich hängt es von diesen Parametern ab, welche innere Festigkeit die Schichten aufweisen, wie sie auf dem Kunststoff haften und folglich, wie die Schichten bei der späteren Durchmischung und Durchknetung des Systems im gesamten Volumen des Kunststoffes dispergiert werden können und welche geometrische Form diese im Kunststoff dispergierten, diskreten Partikel aufweisen, woraus sich dann auch die Elastizitäts- und Festigkeits-Werte des erfindungsgemäß hergestellten Kunststoffes im Vergleich zu den Werten des Ausgangskunststoffes ergeben.

Um den direkten Kontakt von beschichteten Flächen zu verhindern, können unbeschichtete Zwischenfolien verwendet werden. Diese unbeschichteten Zwischenfolien müssen natürlich in die Bilanz "Lagen Beschichtung pro Dickeneinheit Kunststoff" mit einbezogen werden.

Durch die Verwendung unbeschichteter Zwischenfolien wird die Frage der Feuchtigkeitsaufnahme für die folgenden Verfahrensschritte neuerlich wichtig, die durch die doppelseitige Beschichtung der (hygroskopischen) Kunststoff-Folien weitgehend eliminiert worden war. Allerdings ist die für derartige Zwischenfolien benötigte Masse im Verhältnis zur Masse der beschichteten Folie mindestens um den Faktor 2 kleiner. Trotzdem müssen auch die Zwischenfolien auf irgendeine Weise in trockenem Zustand sein, bevor sie, zusammen mit der beschichteten Kunststoff-Folie in einem Prozeß mit (für Kunststoffe) relativ hoher Temperatur verwendet werden.

Es ist eine Frage von vorhandenen Möglichkeiten und technischen wie wirtschaftlichen Erwägungen, ob man lieber dieses zusätzliche, wenn auch guantitativ nicht so große, Problem der Trocknung einer unbeschichteten und daher feuchtigkeitsempfänglichen Zwischenfolie in Kauf nehmen und lösen möchte, oder aber die Vor- und Nachteile von 2-seitig beschichteter Folien ohne Zwischenfolie wählt, oder sich gleich mit nur 1-seitig beschichteten Folien zufrieden gibt.

Eine Möglichkeit Kunststoffe trocken zu verarbeiten, ist die, daß die beschichtete Folie, die zunächst einfach der Umgebungsluft ausgesetzt werden konnte, zusammen mit dieser Zwischenfolie zerkleinert (gehäckselt) und dann gemeinsam getrocknet und in diesem Zustand, aber gesichert gegen die Aufnahme von Feuchtigkeit, verwahrt wird.

Das gehäckselte Produkt wäre bei geeigneten Zufuhreinrichtungen, z.B. "Stopfschnecken", recht gut zur Beladung von Verarbeitungsmaschinen geeignet.
Um aber qualitativ höchstwertige Endprodukte zu erhalten, wird es im allgemeinen besser sein, aus dem getrockneten Häcksel zunächst einmal mittels eines Extruders oder anderer Maschinen, mit welchen dauerthomoplastische Kunststoffe granuliert (pelletiert) werden können (z.B. Theysohn-Compounder, Drais-Gelimat, Pallmann-Plast-Agglomerator oder Condux-Plastcopactor) ein Granulat als Zwischenprodukt herzustellen, das dann, ganz wie allgemein üblich, für die Beladung jeder gängigen Maschine zur Herstellung von Endprodukten verwendet werden kann.
Dieses Granulat mit unbeschichteter Oberfläche ist aber wieder hygroskopisch.
Da es bei der Endverarbeitung auch wieder thermisch belastet wird, ist es vor der Beladung der Endverarbeitungsmaschine im allgemeinen neuerlich zu trocken, was aber nach dem Stand der Technik in dieser Form des Granulates ohnehin leicht möglich und, bei hygroskopischen Kunststoffen, auch schon weitgehend üblich ist.

Im folgenden werden Verfahrensvarianten für die Weiterverarbeitung von mit Wirkstoffen beschichteten Kunststoffen beschrieben.

Es soll dabei unterschieden werden zwischen verschiedenen Trägern, nämlich Folien, Granulat und Fasern.

Folien können in großen Mengen wirtschaftlich in jeder benötigten Breite und, vor allem, in fast jeder benötigten Stärke hergestellt werden. Letztere Möglichkeit ergibt eine sehr breite Variationsmöglichkeit für den Gehalt an Wirkstofffläche je Volumeneinheit, der letztlich die Wirksamkeit des fertigen Kunststoffes bestimmt. In Anbetracht der geringen Kosten je Gewichtseinheit und des geringen Aufwandes für die Beschichtung, für die bei Folien eine ausgereifte, hochautomatisierte Technologie vorhanden ist, ist an die Verwendung von Folien als Rohstoff wohl immer dann zu denken, wenn durchschnittlich große Wirkstoffflächen je Volumeneinheit Kunststoff zu erzeugen sind. Die Schwierigkeiten der Weiterverarbeitung halten sich, bei einer entsprechend großen Fertigung, in Grenzen.

Wird zur Beschichtung einer Folie nur ein Wirkstoff verwendet, sind folgende Ausführungsformen zu unterscheiden:
(a) einseitige Beschichtung der Folie mit nur einem Wirkstoff und Weiterverarbeitung der beschichteten Folie im Stapel oder aufgerollt, so daß jeweils eine Wirkstoffschicht auf einer unbeschichteten Fläche zu liegen kommt, was beim Aufrollen automatisch gegeben ist
(2) zweiseitige Beschichtung der Folie mit nur einem Wirkstoff; dadurch kann bei halbwegs zügiger Vorgangsweise auf eine neuerliche Trocknung vor der Weiterverarbeitung zum Endprodukt verzichtet werden. Diese Verfahrensweise führt aber im Zug der Weiterverarbeitung zu einem Verlust an aktiver Wirkstofffläche, weil bei der Weiterverarbeitung unvermeidbar direkt Wirkstoffflächen aufeinander gepreßt werden
(c) zweiseitige Beschichtung der Folie mit nur einem Wirkstoff, wobei eine unbeschichtete Zwischenfolie verwendet wird, die den teilweisen Verlust von aktiver Fläche im Zug der Weiterverarbeitung dadurch verhindert, daß hierbei Wirkstoffflächen nicht dirket aufeinander gepreßt werden; vielmehr entsteht durch diese Zwischenfolie eine ionen-leitende Zwischenschicht.

Werden zur Beschichtung von Folien 2 verschiedene Wirkstoffe verwendet, sind folgende Ausführungsformen zu unterscheiden:
(a) einseitige Beschichtung der Folien
   (i) einseitige Beschichtung von Folien mit jeweils nur einem der beiden Wirkstoffe; diese Folien werden anschließend durch Stapelung gemischt, wobei das gewünschte Verhältnis der aktiven Flächen der beiden Wirkstoffe zueinander durch die Zahl der jeweiligen Lagen entsprechend beschichteter Folien eingestellt werden kann
   (ii) einseitige Beschichtung der Folien mit den beiden Wirkstoffen simultan in einer Schicht.
      Hierbei kommt es zu keiner echten Legierungsbildung. Das gewünschte Verhältnis der Wirkstoffkonzentration kann im Prinzip durch das Verhältnis des Anteiles jeder der beiden Wirkstoffkomponenten bei der Beschichtung eingestellt werden. Das Verhältnis der Abgabe von Ionen dieser beiden Wirkstoffe bleibt während der Zeit der Anwendung aber nicht stabil.
(b) zweiseitige Beschichtung der Folien
   (i) zweiseitige Beschichtung von Folien mit jeweils nur einem der beiden Wirkstoffe auf Ober- und Unterseite derselben Folie; anschließend werden die Folien durch Stapelung gemischt, wobei das gewünschte Verhältnis der aktiven Flächen der beiden Wirkstoffe zueinander durch die Zahl der jeweiligen Lagen entsprechend beschichteter Folien eingestellt werden kann, oder die Mischung erfolgt durch Zusammenhaspelung ohne Verwendung einer unbeschichteten Zwischenfolie; dies ermöglicht bei halbwegs zügiger Vorgangsweise die Einsparung einer neuerlichen Trocknung vor der Weiterverarbeitung zum Endprodukt.
      Es kommt im Zug der Weiterverarbeitung unvermeidbar dazu, daß Flächen, die mit den beiden Wirksubstanzen beschichtet sind, direkt aufeinandergepreßt werden, was im späteren feuchten Zustand zur Bildung von Lokalelementen führt. Das führt dazu, daß aus dem 2 Wirkstoffe enthaltenden Kunststoff im Verlauf des Gebrauches zunächst bevorzugt Ionen des unedleren Elementes an die Oberfläche kommen, ehe sich ein Endzustand allmählich stabilisiert
   (ii) zweiseitige Beschichtung der Folien mit jeweils nur einem der beiden Wirkstoffe auf beiden Seiten derselben Folie, und nachfolgender Mischung der Folien durch Stapelung, wobei das gewünschte Verhältnis der aktiven Flächen der beiden Wirkstoffe zueinander durch die Zahl der jeweiligen Lagen entsprechend beschichteter Folien eingestellt werden kann, oder Zusammenhaspelung jeweils unter Verwendung einer unbeschichteten Zwischenfolie. Die vorstehend unter Punkt (i) beschriebene Bildung von Lokalelementen und die damit anfangs gegebene stärkere Abgabe von Ionen des unedleren Elementes unterbleibt, weil sich im Zug der Weiterverarbeitung, bedingt durch die unbeschichtete Zwischenfolie, keine Schichten aus verschiedenen Wirksubstanzen aufeinanderpressen können
   (iii) zweiseitige Beschichtung der Folie mit einem der beiden Wirkstoffe auf einer Seite und mit dem anderen der beiden Wirkstoffe auf der anderen Seite der Folie und nachfolgender Stapelung oder Aufhaspelung ohne Verwendung einer unbeschichteten Zwischenfolie; dies ermöglicht bei halbwegs zügiger Vorgangsweise die Einsparung einer neuerlichen Trocknung vor der Weiterverarbeitung zum Endprodukt.
      Es kommt im Zug der Weiterverarbeitung unvermeidbar dazu, daß Flächen, die mit den beiden Wirksubstanzen beschichtet sind, direkt aufeinandergepreßt werden, was im späteren, feuchten Zustand zur Bildung von Lokalelementen führt. Das führt dazu, daß aus dem 2 Wirkstoffe enthaltenden Kunststoff im Verlauf des Gebrauches zunächst bevorzugt Ionen des unedleren Elementes an die Oberfläche kommen, ehe sich ein Endzustand allmählich stabilisiert
   (iv) zweiseitige Beschichtung der Folien mit einem der beiden Wirkstoffe auf einer Seite und mit dem anderen der beiden Wirkstoffe auf der anderen Seite der Folie und nachfolgender Stapelung oder Aufhaspelung unter Verwendung einer unbeschichteten Zwischenfolie: Die vorstehend unter Punkt (i) beschriebene Bildung von Lokalelementen und die damit anfangs gegebene stärkere Abgabe von Ionen des unedleren Elementes unterbleibt, weil sich im Zug der Weiterverarbeitung, bedingt durch die unbeschichtete Zwischenfolie, keine Schichten aus verschiedenen Wirksubstanzen aufeinanderpressen können.

Bei der Verwendung von 3 und mehr Wirkstoffen sind die Prinzipien der für 2 Wirkstoffe erläuterten Ausführungsformen sinngemäß zu übertragen.

Granulat kann in großen Mengen wirtschaftlich hergestellt werden, allerdings nur in einem recht beschränkten Durchmesserbereich. Das Verhältnis von Oberfläche zu Volumen ist hierbei streng (linear) in Abhängigkeit vom Granulatdurchmesser definiert. Diese Eigenschaft ergibt eine sehr kleine Variationsmöglichkeit für den Gehalt an Wirkstofffläche je Volumeneinheit, der letztlich die Wirksamkeit des fertigen Kunststoffes bestimmt.
In Anbetracht der geringen Kosten je Gewichtseinheit und des auch nicht sehr großen Aufwandes für die Beschichtung, wobei vor allem die Technologie der Beschichtung von Schüttgut (z.B. von elektrischen Widerständen) verwendet werden kann (beim erfindungsgemäßen Verfahren kommt es nicht auf eine sehr gleichmäßige Beschichtung an), sowie der angenehmen Weiterverarbeitungsmöglichkeit von Granulat zu Endprodukten, ist an dessen Verwendung als Rohstoff wohl immer dann zu denken, wenn geringe Wirkstoffflächen je Volumeneinheit Kunststoff ausreichen.

Wird zur Beschichtung von Granulat nur ein Wirkstoff verwendet, sind folgende Ausführungsformen zu unterscheiden:
(a) einmalige Beschichtung des Granulates
(b) zweimalige Beschichtung des Granulates mit zwischengeschalteter Durchknetung und erneuter Granulation; im Vergleich zur einmaligen Beschichtung ergibt sich eine doppelte spezifische Wirkstofffläche.
(c) x-malige Beschichtung des Granulates mit jeweils zwischengeschalteter Durchknetung und erneuter Granulation; im Vergleich zur einmaligen Beschichtung ergibt sich eine x-fache spezifische Wirkstofffläche.

Werden zur Beschichtung von Kunststoff-Granulat 2 Wirkstoffe verwendet, sind folgende Ausführungsformen zu unterscheiden:
(a) Beschichtung von getrennten Granulatmengen mit je 1 Wirkstoff wie vorstehend beschrieben und nachfolgende Mischung der mit verschiedenen Wirkstoffen beschichteten Granulate im gewünschten Verhältnis der Wirkstoffe zueinander
(b) Simultane oder sequentielle Beschichtung des Granulates mit verschiedenen Wirkstoffen.
   Hierbei kommt es zu keiner echten Legierungsbildung. Das gewünschte Verhältnis der verschiedenen Wirkstoffe kann durch das Verhältnis des Anteiles jeder der beiden Wirkstoffkomponenten der Beschichtung eingestellt werden. Das Verhältnis der Abgabe von Ionen dieser beiden Wirkstoffe bleibt aber während der Zeit der Anwendung nicht stabil.
(c) Beschichtung von Granulat zuerst mit einem Wirkstoff nach vorstehend genanntem Verfahren, Durchknetung, Granulation und erneute Beschichtung dieses Granulates nach vorstehendem Verfahren mit demselben oder aber mit dem anderen Wirkstoff. Die angestrebte Fläche jedes Wirkstoffes in der Volumeneinheit Kunststoff und damit auch das Verhältnis der beiden Wirkstoffe zueinander kann durch die Zahl der jeweiligen Beschichtungsvorgänge mit neuerlich nachfolgender Granulation eingestellt werden.

Bei der Verwendung von 3 und mehr Wirkstoffen sind die Prinzipien der für 2 Wirkstoffe erläuterten Ausführungsformen sinngemäß auf solche Systeme zu übertragen.

Fasern können in großen Mengen einigermaßen wirtschaftlich hergestellt werden.
Dabei ist ein ziemlich großer Bereich von erzeugbaren Durchmessern möglich, insbesondere in die Richtung zu sehr kleinen Durchmessern. Das ergibt an sich eine große Variationsmöglichkeit für den Gehalt an Wirkstofffläche je Volumeneinheit Kunststoff, welcher letztlich die Wirksamkeit des fertigen Kunststoffes bestimmt.
In Anbetracht der nicht ganz geringen Fertigungskosten je Gewichtseinheit und des beträchtlichen Aufwandes für die Beschichtung wie auch für die Weiterverarbeitung ist an die Verwendung von Fasern als Rohstoff wohl nur dann zu denken, wenn extrem große Wirkstoffflächen je Volumeneinheit Kunststoff zu erzeugen sind.

Bei der Beschichtung von Fasern mit einem Wirkstoff sind folgende Ausführungsformen zu unterscheiden:
(a) einmalige Beschichtung der Fasern ermöglicht bei wasserdampfdichter Beschichtung rundherum die Einsparung einer neuerlichen Trocknung vor der Weiterverarbeitung bzw. Endverarbeitung, führt aber im Zug dieser Weiter- bzw. Endverarbeitung zu einem Verlust an aktiver Wirkstofffläche (im Verhältnis zur rein rechnerisch zu erwartenden, aktiven Fläche im Endprodukt), weil dabei unvermeidbar direkt Wirkstoffflächen aufeinander gepreßt werden.
(b) zweimalige Beschichtung der Fasern mit zwischengeschalteter Durchknetung und erneuter Verspinnung ergibt die doppelte spezifische Wirkstofffläche im Vergleich zur einmaligen Beschichtung
(c) x-malige Beschichtung der Fasern mit jeweils zwischengeschalteter Durchknetung und erneuter Verspinnung ergibt die x-fache spezifische Wirkstofffläche im Vergleich zur einmaligen Beschichtung.

Bei der Beschichtung von Fasern mit 2 Wirkstoffen sind die folgenden Ausführungsformen zu unterscheiden:
(a) Beschichtung von getrennten Fasermengen mit je 1 Wirkstoff wie vorstehend beschrieben und nachfolgende Mischung der mit verschiedenen Wirkstoffen beschichteten Fasern im gewünschten Verhältnis der Wirkstoffe zueinander
(b) Simultane oder sequentielle Beschichtung von Fasern mit verschiedenen Wirkstoffen, wobei es zu keiner echten Legierungsbildung kommt. Das gewünschte Verhältnis der Wirkstoffkonzentrationen kann im Prinzip durch das Verhältnis des Anteiles jeder der beiden Wirkstoffkomponenten bei der Beschichtung eingestellt werden.
   Das Verhältnis der Abgabe von Ionen dieser beiden Wirkstoffe bleibt aber während der Zeit der Anwendung nicht stabil
(c) Beschichtung von Fasern zuerst mit einem Wirkstoff nach vorstehend beschriebenen Verfahren, Durchknetung, erneute Verspinnung und Beschichtung dieser Fasern nach vorstehenden Verfahren neuerlich mit demselben oder aber mit dem anderen Wirkstoff.
   Die angestrebte Fläche jedes Wirkstoffes in der Volumeneinheit Kunststoff und damit auch das Flächenverhältnis der beiden Wirkstoffe zueinander kann durch die Zahl der jeweiligen Beschichtungsvorgänge mit neuerlich nachfolgender Verspinnung eingestellt werden.

Bei der Verwendung von 3 und mehr Wirkstoffen sind die Prinzipien der für 2 Wirkstoffe erläuterten Ausführungsformen sinngemäß auf solche Systeme zu übertragen.

Eine Sonderform von Folien stellen Bänder dar. Diese können in großen Mengen wirtschaftlich in jeder benötigten Breite und Stärke hergestellt werden. Letztere Möglichkeit ergibt eine sehr breite Variationsmöglichkeit für den Gehalt an Wirkstofffläche je Volumeneinheit, der letztlich die Wirksamkeit des fertigen Kunststoffes bestimmt.
In Anbetracht der geringen Fertigungskosten je Gewichtseinheit und des relativ geringen Aufwandes für die auch 2-seitige Beschichtung von Bändern, bei denen die bei Folien ausgereifte, hochautomatisierte Technologie, angewendet werden kann, ist an die Verwendung von Bändern als Rohstoff wohl immer dann zu denken, wenn eine durchschnittlich große Wirkstofffläche je Volumeneinheit Kunststoff zu erzeugen sind.
Bänder können auch an den Schmalseiten ausreichend beschichtet werden, so daß sie nachher als ringsherum beschichtet gelten können.
Anders als breite Folien können Bänder aber ohne weitere Vorzerkleinerung (und den normalerweise darauf folgenden, weiteren Zwischenschritten) in viele Extrusionsmaschinen direkt eingezogen werden, was eine entscheidende Vereinfachung des gesamten Fertigungsverfahrens erfindungsgemäßer Kunststoffe erbringt, die freilich mit einem erhöhten Einsatz an Wirkstoff erkauft werden muß.

Die zweiseitige Beschichtung der Folie mit nur einem Wirkstoff ermöglicht bei wasserdampfdichter Beschichtung rundherum die Einsparung einer neuerlichen Trocknung vor der Weiterverarbeitung bzw. Endverarbeitung, führt aber im Zug dieser Weiter- bzw. Endverarbeitung zu einem Verlust an aktiver Wirkstofffläche im Endprodukt, weil dabei unvermeidbar direkt Wirkstoffflächen aufeinander gepreßt werden.

Eine weitere Ausführungsform ist dadurch gekennzeichnet, daß zunächst ein beschichteter Kunststoffrohling nach einem der vorstehend beschriebenen Verfahren hergestellt wird, jedoch mit einer höheren Konzentration an einem oder mehreren antimikrobiell wirksamen Metall(en) und/oder Metallverbindung(en); dieser beschichtete Kunststoffrohling wird anschließend zusammen mit unbeschichteten Kunststoffrohlingen zerkleinert und/oder eingeschmolzen und schließlich nach einem üblichen Verfahren in die gewünschte Form gebracht. Bei diesem Verfahren kann für die unbeschichteten Kunststoffrohlinge der gleiche und/oder andere Kunststoff(e) verwendet werden als für den beschichteten Kunststoffrohling.

### Beispiel

In einer für die Entspiegelung von optischen Gläsern bestimmten Hochvakuum-Anlage wurden ca. 80 cm² große Folien von 0,25 mm Dicke aus Polyurethan mit einer Silberschicht von etwa 10 nm Dicke bedampft. Die Silberschicht zeigte lichtmikroskopisch keinerlei Eigenstruktur, es waren nur die Unebenheiten der Folie als Unebenheiten der Schicht wiederzuerkennen. Die Folien wurden danach zerkleinert und unter Umrühren bei ca. 240°C eingeschmolzen. Daraus wurden durch Heißpressen Prüfkörper (kleine Plättchen) hergestellt.

Der Silbergehalt dieser aus den bedampften Silberfolien hergestellten Prüfkörper wurde nach dem Aufschluß mit Salpetersäure mittels der AAS-Methode zu etwa 350 ppm bestimmt, wobei allerdings, bedingt durch Unvollkommenheiten der Zubereitung der Proben, erhebliche Streuungen zwischen den (drei) ermittelten Meßwerten hingenommen werden mußten. Größenordnungsmäßig liegt dieses Ergebnis bei nur 1/30 von jenen Werten, wie sie für die wirkungsvolle Verwendung von Silberpulver im Stand der Technik als mindestens nötig angegeben werden.

Diese Prüfkörper zeigten sich als antimikrobiell gegen die Besiedelung mit dem Bakterium Staphylokokkus epidermidis voll wirksam. Diese Untersuchung wurde gemäß dem in DE-C-42 26 810 beschriebenen Verfahren durchgeführt.

### Test auf antimikrobielle Wirksamkeit

Die verwendeten Kunststoffproben sind per Hand durch Einschmelzen und Verrühren unter kontrollierten Temperaturverhältnissen gefertigt worden.

Die Proben in Form von Platten mit 132 mm Durchmesser haben in der Phase des Erkaltens jeweils 8 napfförmige Vertiefungen mit einem Volumen von jeweils 0,5 ml eingeprägt bekommen.

Es sind insgesamt 9 verschiedene Probeplatten aus dauerthermoplastischen Polyurethan-Folien "Platilon U 073", (Polyether-Typ) 0,18 mm dick, hergestellt worden, die in einer eigentlich für die Beschichtung optischer Teile bestimmten Anlage mit
a) Silber 10 nm einseitig,
b) Silber 30 nm einseitig,
c) Silber 60 nm einseitig,
d) Silber 30 nm zweiseitig,
e) Silber 30 nm auf der einen Seite und Silber 60 nm auf der anderen Seite
f) Silber 30 nm auf der einen Seite und Kupfer 30 nm auf der anderen Seite
g) Kupfer 30 nm zweiseitig,
beschichtet worden waren. Dabei sind zu Kontrollzwecken die Versuchsplatten a) und c) in 2-facher Ausfertigung hergestellt worden.

Zu Vergleichszwecken wurden 2 Platten aus unbeschichteten Folien hergestellt.

Nach der üblichen Gas-Sterilisation dieser Platten wurden die Vertiefungen mit physiologischer Kochsalzlösung gefüllt und in aufeinanderfolgenden Untersuchungsschritten mit folgenden Keimen in den angegebenen Konzentrationen beimpft:
Staphylococcus epidermidis: 5 x 10⁷ Keime/ml
Staphylococcus aureus: 5 x 10⁷ Keime/ml
Streptococcus faecalis: 5 x 10⁷ Keime/ml
Escherichia coli: 5 x 10⁷ Keime/ml
Pseudomonas aeruginosa: 5 x 10⁷ Keime/ml
Candida albicans: 5 x 10⁷ Keime/ml

Diese so mit Keimsuspension belasteten Platten wurden sodann in einen Brutschrank eingebracht und 48 Stunden darin belassen.

Nach etwa einer halben Stunde ist, visuell beurteilt, der Inhalt der Näpfe eingetrocknet.

Nach insgesamt 48 Stunden wurden die Platten dem Brutschrank entnommen und die Näpfe wieder mit physiologischer Kochsalzlösung aufgefüllt. Nach weiteren 15 Minuten wurde die neu entstandene Suspension entnommen und in Nährbouillon eingebracht.

Während alle Keimsuspensionen, die auf Platten aus Kunststoff im Ausgangszustand aufgebracht worden waren, nach diesem Versuch sofort wieder voll proliferierten, waren bei allen Kunststoffplatten, die nach den Verfahren a) bis f) behandelt waren, grundsätzlich alle der getesteten Keime abgestorben.
Suspensionen, in denen sich doch noch enige Keime als vermehrungsfähig erwiesen, ergaben sich nur bei den Näpfen, bei denen man bei visueller Kontrolle deutliche "blinde Flecken" erkennen konnte, d.h. aufgrund von Unvollkommenheiten des händischen Verrührens ist unter der Oberfläche dieser "blinden Flecke" kein Metall zu sehen. Das Vorhandensein von vermehrungsfähigen Keimen ist also nicht der Wirkung des erfindungsgemäß hergestellten Kunststoffes zuzuordnen, sondern ausschließlich Unvollkommenheiten bei dessen Herstellung.

Die durchgeführten Versuche, bei denen keine "blinden Flecke" zu Ausreißern geführt haben, haben einen 100%igen Erfolg erbracht.

Dieses Ergebnis belegt den Erfindungsgedanken, daß
- neben der Wahl des Wirkstoffes und auch der
- Wahl des Kunststoffes, hier vor allem bezüglich seiner Fähigkeit, Wasser aufzunehmen und Metallionen die Diffusion zu ermöglichen,
vor allem
- das Verhältnis von aktiver Oberfläche pro Volumeneinheit Kunststoff
über die Wirksamkeit entscheidet und nicht der prozentuale Gewichtsanteil von Wirkstoff im Kunststoff entscheidend ist, zumal ja in allen untersuchten Fällen dieser Anteil weit geringer ist, als er gemäß vorliegender Patentliteratur für eine nachweisbare Wirksamkeit mindestens sein sollte.

## Patentansprüche

1. Verfahren zur Herstellung von Kunststoffkörpern, dadurch gekennzeichnet, daß ein Kunststoffrohling mit einem(r) oder mehreren antimikrobiell wirksamen Metall(en) und/oder Metallverbindung(en) mittels eines chemischen oder physikalischen Verfahrens beschichtet wird, der beschichtete Kunststoffrohling danach zerkleinert und/oder eingeschmolzen und anschließend nach einem üblichen Verfahren in die gewünschte Form gebracht wird.

2. Verfahren gemäß Anspruch 1, dadurch gekennzeichnet, daß der Rohling in Form von Folien vorliegt.

3. Verfahren gemäß Anspruch 1, dadurch gekennzeichnet, daß der Rohling in Form von Granulat vorliegt.

4. Verfahren gemäß Anspruch 1, dadurch gekennzeichnet, daß der Rohling in Form von Fasern vorliegt.

5. Verfahren gemäß einem der Ansprüche 2 bis 4, dadurch gekennzeichnet, daß der Rohling mit einem(r) antimikrobiell wirksamen Metall oder Metallverbindung beschichtet wird.

6. Verfahren gemäß einem der Ansprüche 2 bis 4, dadurch gekennzeichnet, daß der Rohling mit zwei oder mehreren verschiedenen antimikrobiell wirksamen Metallen oder Metallverbindungen beschichtet wird.

7. Verfahren gemäß Anspruch 2, 5 und 6, dadurch gekennzeichnet, daß die Folie auf einer Seite beschichtet wird.

8. Verfahren gemäß Anspruch 2, 5 und 6, dadurch gekennzeichnet, daß die Folie auf beiden Seiten beschichtet wird.

9. Verfahren gemäß Anspruch 7, dadurch gekennzeichnet, daß eine Folie mit 2 oder mehreren antimikrobiell wirksamen Metallen oder Metallverbindungen simultan auf einer Seite beschichtet wird.

10. Verfahren gemäß Anspruch 8, dadurch gekennzeichnet, daß Ober- und Unterseite einer Folie jeweils mit verschiedenen antimikrobiell wirksamen Metallen oder Metallverbindungen beschichtet werden.

11. Verfahren gemäß Anspruch 3 und 5, dadurch gekennzeichnet, daß Granulat mindestens zweimal beschichtet wird.

12. Verfahren gemäß Anspruch 3 und 6, dadurch gekennzeichnet, daß dasselbe Granulat simultan oder sequentiell mit mindestens 2 verschiedenen antimikrobiell wirksamen Metallen oder Metallverbindungen beschichtet wird.

13. Verfahren gemaß Anspruch 4 und 5, dadurch gekennzeichnet, daß die Fasern mindestens zweimal beschichtet werden.

14. Verfahren gemäß Anspruch 4 und 6, dadurch gekennzeichnet, daß dieselben Fasern simultan oder sequentiell mit mindestens 2 verschiedenen antimikrobiell wirksamen Metallen oder Metallverbindungen beschichtet werden.

15. Verfahren gemäß Anspruch 8, dadurch gekennzeichnet, daß (beidseitig beschichtete) Folien unter Verwendung von unbeschichteten Zwischenfolien für die Weiterverarbeitung gestapelt oder zusammengehaspelt werden.

16. Verfahren gemäß einem der Ansprüche 1 bis 15, dadurch gekennzeichnet, daß zunächst ein beschichteter Kunststoffrohling gemäß einem in den Ansprüchen 1 bis 15 beanspruchten Verfahren, jedoch mit einer höheren Konzentration als der gewünschten Endkonzentration an einem(r) oder mehreren antimikrobiell wirksamen Metall(en) und/oder Metallverbindung(en), hergestellt wird, dieser anschließend zusammen mit einem oder mehreren unbeschichteten Kunststoffrohlingen zerkleinert und/oder eingeschmolzen und anschließend nach einem üblichen Verfahren in die gewünschte Form gebracht wird.

17. Verfahren gemäß einem der Ansprüche 1-16, dadurch gekennzeichnet, daß die Beschichtung mittels plasmaunterstützter Bedampfung erfolgt.

18. Kunststoffkörper, erhältlich gemäß einem der vorstehend beanspruchten Verfahren.

## Claims

1. Process for producing plastic bodies, characterized in that a plastic blank is coated with one or more antimicrobially active metal(s) and/or metal compound(s) by means of a chemical or physical process, the coated blank is then ground and/or molten and finally imparted the desired shape by means of a common process.

2. The process according to claim 1, characterized in that the blank is present in the form of films.

3. The process according to claim 1, characterized in that the blank is present in the form of granulate.

4. The process according to claim 1, characterized in that the blank is present in the form of fibers.

5. The process according to any one of claims 2 to 4, characterized in that the blank is coated with one antimicrobially active metal or metal compound.

6. The process according to any of claims 2 to 4, characterized in that the blank is coated with two or more antimicrobially active metals or metal compounds.

7. The process according to claims 2, 5 and 6, characterized in that the film is coated on one side.

8. The process according to claims 2, 5 and 6, characterized in that the film is coated on both sides.

9. The process according to claim 7, characterized in that a film is simultaneously coated on one side with two or more antimicrobially active metals or metal compounds.

10. The process according to claim 8, characterized in that the top side and the bottom side of a film are each coated with different antimicrobially active metals or metal compounds.

11. The process according to claims 3 and 5, characterized in that the granulate is coated at least twice.

12. The process according to claims 3 and 6, characterized in that the same granulate is coated simultaneously or sequentially with at least two different antimicrobially active metals or metal compounds.

13. The process according to claims 4 and 5, characterized in that the fibers are coated at least twice.

14. The process according to claims 4 and 6, characterized in that the same fibers are coated simultaneously or sequentially with at least two different antimicrobially active metals or metal compounds.

15. The process according to claim 8, characterized in that films (coated on both sides) are stacked or reeled for further processing together with uncoated intermediate films.

16. The process according to any one of claims 1 to 15, characterized in that first a coated plastic blank is produced by means of a process according to any one of claims 1 to 15, however, with a concentration of one or more antimicrobially active metal(s) and/or metal compound(s) higher than the desired final concentration, the blank is then ground and/or molten together with one or more uncoated plastic blanks and finally imparted the desired shape by means of a common process.

17. The process according to any one of claims 1 to 16, characterized in that coating is carried out by means of plasma-supported vapor deposition.

18. Plastic body, obtainable by one of the preceding processes.

## Revendications

1. Procédé pour la fabrication de corps en matière plastique, caractérisé en ce qu'une ébauche en matière plastique est revêtue au moyen d'un procédé physique ou chimique, d'un ou de plusieurs métaux et/ou composés métalliques exerçant une action antimicrobienne, l'ébauche en matière plastique revêtue est ensuite fractionnée et/ou fondue, et est enfin amenée à la forme souhaitée par un procédé habituel.

2. Procédé selon la revendication 1, caractérisé en ce que l'ébauche présente la forme de feuilles.

3. Procédé selon la revendication 1, caractérisé en ce que l'ébauche présente la forme d'un granulat.

4. Procédé selon la revendication 1, caractérisé en ce que l'ébauche présente la forme de fibres.

5. Procédé selon l'une des revendications 2 à 4, caractérisé en ce que l'ébauche est revêtue d'un métal ou d'un composé métallique exerçant une action antimicrobienne.

6. Procédé selon l'une des revendications 2 à 4, caractérisé en ce que l'ébauche est revêtue de deux ou plusieurs métaux ou composés métalliques différents exerçant une action antimicrobienne.

7. Procédé selon les revendications 2, 5 et 6, caractérisé en ce que la feuille est revêtue sur une face.

8. Procédé selon les revendications 2, 5 et 6, caractérisé en ce que la feuille est revêtue sur les deux faces.

9. Procédé selon la revendication 7, caractérisé en ce qu'une feuille est revêtue simultanément sur une face de 2 ou de plusieurs métaux ou composés métalliques exerçant une action antimicrobienne.

10. Procédé selon la revendication 8, caractérisé en ce que la face supérieure et la face inférieure d'une feuille sont chacune revêtues de différents métaux ou composés métalliques exerçant une action antimicrobienne.

11. Procédé selon les revendications 3 et 5, caractérisé en ce que le granulat est recouvert au moins deux fois.

12. Procédé selon les revendications 3 et 6, caractérisé en ce que le même granulat est revêtu simultanément ou séquentiellement d'au moins 2 différents métaux ou composés métalliques exerçant une action antimicrobienne.

13. Procédé selon les revendications 4 et 5, caractérisé en ce que les fibres sont revêtues au moins deux fois.

14. Procédé selon les revendications 4 et 6, caractérisé en ce que les mêmes fibres sont revêtues simultanément ou séquentiellement d'au moins 2 différents métaux ou composés métalliques exerçant une action antimicrobienne.

15. Procédé selon la revendication 8, caractérisé en ce que des feuilles (revêtues sur les deux faces) sont empilées ou enroulées ensemble pour la poursuite de la transformation, en utilisant des feuilles intermédiaires non revêtues.

16. Procédé selon l'une des revendications 1 à 15, caractérisé en ce qu'une ébauche en matière plastique revêtue est d'abord préparée par un procédé selon l'une des revendications 1 à 15, mais à une concentration plus élevée que la concentration finale souhaitée en un ou plusieurs métaux ou composés métalliques exerçant une action antimicrobienne, celle-ci est ensuite fractionnée et/ou fondue en même temps qu'une ou plusieurs ébauches en matière plastique non revêtues, et est enfin amenée à la forme voulue par un procédé habituel.

17. Procédé selon l'une des revendications 1 à 16, caractérisé en ce que le revêtement s'effectue au moyen d'une vaporisation à l'aide d'un plasma.

18. Corps en matière plastique obtenus au moyen d'un procédé selon l'une des revendications précédentes.
